# EUROPÄISCHE PATENTSCHRIFT

(11) **EP 2 497 433 B1**
(45) Veröffentlichungstag und Bekanntmachung des Hinweises auf die Patenterteilung: **13.07.2016**
(21) Anmeldenummer: 12158178.9
(22) Anmeldetag: 06.03.2012
(51) Int. Cl.: A61B 17/34, A61B 17/00

(54) **Mehrfachtrokarsystem**
Multiple trocar system
Système de trocart multiple

(30) Priorität: 07.03.2011 DE 102011013889
(43) Veröffentlichungstag der Anmeldung: 12.09.2012
(73) Patentinhaber: Karl Storz GmbH & Co. KG, 78532 Tuttlingen (DE)
(72) Erfinder: Burghardt, Jens, 15569 Woltersdorf (DE); Bogenschütz, Jürgen, 88499 Altheim (DE); Weinmann, Daniel, 78606 Seitingen- Oberflacht (DE)
(74) Vertreter: Witte, Weller & Partner Patentanwälte mbB

(56) Entgegenhaltungen:
- EP-A2- 2 163 217
- WO-A1-2012/014656
- WO-A2-2007/140449
- US-A- 3 299 883

## Beschreibung

Die Erfindung betrifft einen Trokardorn mit einem langerstreckten schaftartigen Dornkörper, dessen distales Ende mit einer Spitze versehen ist, und dessen proximales Ende einen Kopf aufweist, wobei am Kopf zumindest ein zweiter langerstreckter schaftartiger Dornkörper derart angebracht ist, dass sich die Dornkörper in einem Abstand (A) einander vom Kopf wegerstrecken, wobei der Kopf eine proximale Deckfläche aufweist, und sich die schaftartigen Dornkörper vom distalen Ende des Kopfes wegerstrecken.

Ein derartiger Trokardorn ist aus der US 3 299 833 A bekannt. Der Kopf besteht aus einem langerstreckten etwa stabförmigen Körper, von dessen beiden gegenüberliegenden Ende gelenkig, somit schwenkbar, sich zwei langerstreckte schaftartige Dornkörper wegerstrecken.

Etwa mittig, zwischen den beiden endseitigen Dornkörpern ist noch ein dritter Dornkörper vorhanden. Dieser mittige dritte Dornkörper ist durch eine hülsenartige Öffnung im Kopf hindurchgeführt und erstreckt sich proximalseitig etwas über den Kopf hinaus. An dessen äußeren Ende ist eine etwa scheibenförmige Handhabe vorhanden.

Aus der EP 2 163 217 A2 ist ein minimal-invasives Zugangsgerät bekannt, das einen distalseitigen etwa hohlzylindrischen Abschnitt aufweist, der sich am proximalen Ende konisch aufweitet. Durch den relativ durchmessergroßen zylindrischen Hohlschaft können, nach Setzen des Portes im Körper, mehrere schaftartige Kanülen oder Instrumente durchgeschoben werden.

Die WO 2007/140449 A2 zeigt zwei miteinander verbindbare Teiltrokare in die, nach Verbinden, zwei Trokardorne eingeschoben sind, so dass sich in diesem zusammengesetzten Zustand zwei langerstreckte schaftartige Dornkörper von einem Kopf des Trokardornes wegerstrecken.

Ein Trokar besteht aus einer Trokarhülse und einem darin einzuschiebenden Trokardorn. Der Trokardorn ist so bemessen, dass er den Innenraum der Trokarhülse füllt, und dass dessen Spitze distalseitig über die Trokarhülse hinausreicht. Dabei ist es bekannt geworden, der Spitze des Torkardornes unterschiedliche Geometrien zu verleihen, beispielsweise eine stumpfe Spitze, eine kegelartige Spitze oder eine Dreikantspitze.

Der Zusammenbau aus Trokarhülse und Trokardorn, somit der Trokar, dient dazu, bei der minimal invasiven Chirurgie einen Zugang zu einer inneren Körperhöhle zu schaffen.

Ein weit verbreitetes Anwendungsgebiet ist dabei die Laparoskopie.

Bei der Laparoskopie wird eine Inzision von etwa 1 bis 2 cm Länge in die Haut der Bauchdecke eingebracht. An diese Inzision wird der Trokar über die über die Trokarhülse vorstehende Spitze des Trokardornes angesetzt. Daraufhin wird der Zusammenbau durch die Bauchdecke hindurchgedrückt bis dessen distales Ende in den Bauchraum hineinragt. Anschließend wird der Trokardorn abgezogen und abgelegt.

Die hohle Trokarhülse steckt nunmehr im Körper, also beispielsweise in der Bauchdecke und es kann dann durch die Trokarhülse hindurch ein minimal invasiver Eingriff durchgeführt werden.

Der Außendurchmesser einer Trokarhülse reicht bis etwa 25 mm, so dass letztendlich der Raum, der zum Durchführen von Instrumenten durch die Trokarhülse hindurch relativ klein ist.

Bei minimal invasiven Eingriffen ist es üblich geworden, insbesondere bei der Laparoskopie, mehrere solche Trokarhülsen zu setzen. Dadurch können dann über die mehreren Trokarhülsen verschiedene medizinische Instrumente eingeführt werden, beispielsweise reine Beobachtungsinstrumente, z.B. Endoskope, arbeitende medizinische Instrumente wie Zangen, Scheren, Stanzen und dergleichen und Instrumente zum Zuführen von Medien, beispielsweise gasförmigen Medien zum Aufblähen des Bauchraums oder Spülflüssigkeiten zum Abspülen von insbesondere Blut aus dem Operationsraum.

Es hat sich bei manchen Operationstechniken herausgestellt, dass es günstig ist, wenn zwei benachbarte Trokare in einem ganz definierten Abstand zueinander in den Körper gesetzt werden. Dies insbesondere, wenn mit einem Beobachtungsinstrument, das durch eine erste Trokarhülse eingeschoben wird, ganz gezielt der Arbeitsbereich eines anderen Instruments, das durch eine zweite Trokarhülse durchgeschoben wird, beobachtet werden soll.

Es verlangt von dem Operateur eine erhebliche Erfahrung, zwei oder mehrere Trokare in einem exakt definierten Abstand voneinander zu setzen.

Es ist daher Aufgabe der vorliegenden Erfindung hier Abhilfe zu schaffen und einen Trokar dahingehend weiter zu entwickeln, dass mehrere Trokare ergonomisch in einem definierten Abstand platziert werden können.

Erfindungsgemäß wird die Aufgabe dadurch gelöst, dass der Kopf, auf die proximale Deckfläche gesehen, als abgerundeter Körper ausgebildet ist, und dass ein Umfangsprofil der Deckfläche einer Hüllkurve entspricht, die die Dornkörper mit unterschiedlichem Durchmesser umrundet.

Im einfachsten Falle ist ein zweiter langerstreckter schaftartiger Dornkörper vorgesehen, so dass daraus quasi ein Doppeltrokardorn resultiert, bei dem die beiden schaftartigen Dornkörper an ein und demselben Kopf angeordnet sind und sich von diesem weg erstrecken. Prinzipiell ist es möglich, auch mehr als zwei Dornkörper an einem Kopf anzuordnen.

Dadurch ist es möglich, diese schaftartigen Dornkörper in einer ganz bestimmten Ausrichtung, meist parallel zueinander und in einem ganz definierten Abstand zueinander anzuordnen.

Über jeden der schaftartigen Dornkörper kann nun eine Trokarhülse geschoben werden und der daraus resultierende Trokar ist dann ein Zusammenbau aus einem einzigen Kopf, von dem sich mehrere schaftartige Dornkörper weg erstrecken, auf denen jeweils eine Trokarhülse aufgeschoben ist.

Dieser kompakte Zusammenbau kann beispielsweise bei der Laparoskopie an den entsprechenden Inzisionen an der Bauchdecke angesetzt und in einem einzigen Vorgang durch die Bauchdecke hindurchgeschoben werden. Ein weiterer Vorteil besteht darin, dass der damit zwangsläufig relativ große Kopf des Trokardornes als "Drücker" fungieren kann, über den die Hand des Operateurs die Kraft auf den Trokar überträgt, die notwendig ist, um diesen durch die Bauchdecke hindurchzutreiben.

Nach dem Setzen kann dann der Trokardorn von den entsprechend gesetzten Trokarhülsen, seien es nun zwei, drei oder mehrere, in einem Vorgang abgezogen werden.

Die nunmehr im Körper verbleibenden Trokarhülsen sind zum einen exakt ausgerichtet und weisen insbesondere den gewünschten definierten Abstand voneinander auf.

Dadurch ist der minimal invasive Eingriff, der durch die mehreren Trokarhülsen hindurch durchgeführt wird, wesentlich erleichtert. Ein weiterer Vorteil besteht auch darin, dass ein solcher Trokar, der einen erfindungsgemäßen Trokardorn mit mehreren schaftartigen Dornkörpern aufweist, ein relativ kompaktes und schlankes Gebilde darstellt, das in bereits vorhandene Körperhöhlen zunächst einmal eingeführt werden kann und erst dann weiter innen in der Körperhöhle durch das Gewebe in weiter innen liegende Körperhöhlen eingetrieben wird.

Dies ist beispielsweise bei Operationstechniken im Bereich des weiblichen Unterkörpers sehr vorteilhaft, dort kann der Trokar mit den mehreren Trokarhülsen zunächst durch die Vagina eingeschoben und erst im Bereich der Gebärmutter oder beispielsweise des Douglas-Raumes in die innere Körperhöhle durch das Gewebe hindurchgetrieben werden.

Dies ist wesentlich einfacher und in einem einzigen Vorgang durchzuführen, als nach und nach mehrere einzelne Trokare durch die Vagina bis zu diesen innenliegenden Gewebebereichen zu führen. Letztendlich ist somit das Setzen der Trokare auch wesentlich atraumatischer für eine Patientin durchzuführen.

Der Kopf weist eine proximale Deckfläche auf und die schaftartigen Dornkörper erstrecken sich vom distalen Ende des Kopfes weg.

Diese Maßnahme hat den Vorteil, dass diese proximale Deckfläche als Ansatzfläche, beispielsweise für eine Hand des Operateurs dienen kann, um die notwendige Kraft zum Durchschieben des Trokars durch das Gewebe hindurch auszuüben.

Gemäß der Erfindung ist der Kopf, auf die proximale Deckfläche gesehen, als abgerundeter Körper ausgebildet.

Diese Maßnahme hat den Vorteil, dass der Kopf besonders ergonomisch, also ohne Ecken und Kanten ausgebildet ist, so dass dieser für den Operateur besonders ergonomisch ergriffen werden kann.

Gemäß der Erfindung entspricht ein Umfangsprofil der Deckfläche einer die Dornkörper umhüllenden Konturlinie.

Diese Maßnahme hat den Vorteil, dass auch hier wieder ein besonders ergonomischer abgerundeter Körper geschaffen ist. Zugleich resultiert bei Trokardornen mit Dornen unterschiedlichen Durchmessers eine jeweils von der Außenseite her ersichtliche Konturlinie, die dem Operateur anzeigt, an welcher Seite nun der Trokardorn mit größerem Durchmesser und an welcher Seite der Trokardorn mit dem geringeren Durchmesser angeordnet ist.

Greift man auf das vorher erwähnte Beispiel der transvaginalen Einführung zurück, so ist kurz vor Einstecken des Trokares in das Gewebe schon ein erheblicher Abschnitt des langerstreckten Körpers in die Vagina eingeschoben und somit von außen durch den Operateur kaum oder nur schwer ersichtlich. Diese besondere Konturlinie gibt ihm nun eine Orientierung, wo am Kopf der Trokardorn mit dem größeren Durchmesser und wo der mit dem geringeren Durchmesser sitzt. Entsprechendes gilt, wenn man beispielsweise drei solcher schaftartigen Dornkörper vorsieht, und man dann die Hüllkurve beispielsweise als ein abgerundetes Dreieck oder als einen Art V- oder nierenförmigen Körper ausgestaltet, der entsprechend abgerundet ist. Auch hier hat der Operateur immer eine Orientierung, an welcher Stelle sich die Dorne wegerstrecken, auch wenn er sie kaum einsieht.

In einer weiteren Ausgestaltung der Erfindung weisen die schaftartigen Dornkörper unterschiedliche Durchmesser auf.

Diese Maßnahme hat den Vorteil, dass für einen Operationstyp geeignete unterschiedliche Trokarhülsen mit unterschiedlichem Durchmesser gleichzeitig gesetzt werden können. So kann beispielsweise eine relativ durchmessergroße Trokarhülse gesetzt werden, durch die relativ bulkige arbeitende Instrumente hindurchgeschoben werden und zugleich eine durchmessergeringere relativ schlanke Trokarhülse gesetzt werden, durch die nur ein Beobachtungsinstrument, z.B. ein Endoskop hindurchgeschoben wird.

Das erlaubt eine hohe Flexibilität und ein besonders atraumatisches Setzen von Trokarhülsen unterschiedlichen Durchmessers.

In einer weiteren Ausgestaltung der Erfindung ist zumindest ein schaftartiger Dornkörper lösbar am Kopf angebracht.

Diese Maßnahme hat nun den erheblichen Vorteil, dass ein vorhandener Dornkörper durch einen anderen ersetzt werden kann. Dies ist beispielsweise vorteilhaft, wenn Dornkörper mit unterschiedlichen Längen eingesetzt werden sollen. So ist die Eindringtiefe bei einer fülligen erwachsenen Person bei der Laparoskopie anders als beispielsweise bei einem schlanken Kind. Es können aber nicht nur Dornkörper unterschiedlicher Länge sondern auch unterschiedlicher Spitzengeometrien, wie sie eingangs erwähnt worden sind, eingesetzt werden, je nachdem wie das für den Eingriff am günstigsten ist. Es können auch prinzipiell Trokare unterschiedlichen Schaftdurchmessers ausgewechselt werden, wobei dann entsprechende Vorkehrungen getroffen werden müssen, dass auch diese Wechseldornkörper fest am Kopf sitzen.

In einer weiteren Ausgestaltung der Erfindung ist der zumindest eine lösbare Dornkörper mit dem Kopf über eine Verrastung lösbar verbindbar, wobei am Kopf ein Betätigungselement angeordnet ist, durch das die Verrastung zwischen Kopf und Dornkörper lösbar ist.

Diese Maßnahme hat die Vorteile, dass der Auswechselvorgang einfach durchzuführen ist, und dass aufgrund der Verrastung der Dornkörper ausreichend fest und unverrückbar am Kopf sitzt. Dabei gibt es verschiedene konstruktive Möglichkeiten wie beispielsweise Kugelrasten oder dergleichen, wie sie im Instrumentenbau zur lösbaren Verbindung zwischen einem solchen schaftartigen Körper und einem Basisbauteil eingesetzt werden.

In einer weiteren Ausgestaltung der Erfindung ist am Kopf ein Verstellmechanismus angeordnet, über den der Abstand zwischen benachbarten Dornkörpern veränderbar ist.

Diese Maßnahme hat nun den erheblichen Vorteil, dass auch noch der Abstand zwischen den am Kopf angebrachten Dornkörpern verändert werden kann, so dass besonders flexibel auf entsprechende Gegebenheiten angepasst werden kann.

In Kombination mit der Ausgestaltung des lösbaren Dornkörpers sind nun eine große Vielzahl an Ausgestaltungen und Gebilden erzielbar, die alle an ein und demselben Kopf des Trokardorns angebracht sind. Das können Trokardorne mit unterschiedlich ausgestalteten Spitzengeometrien, unterschiedlicher Länge, unterschiedlichen Durchmessers, unterschiedlicher Schaftgeometrie oder dergleichen sein.

In einer weiteren Ausgestaltung der Erfindung sind an der Seite des Kopfes, von der die schaftartigen Dornkörper vorstehen, Anschläge angeordnet, die eine Einschubtiefe des jeweiligen Dornkörpers in eine Trokarhülse begrenzen.

Diese Maßnahme hat den Vorteil, dass entweder der Trokardorn in die Trokarhülse soweit eingeschoben oder umgekehrt die Trokarhülse auf den Trokardorn nur soweit aufgeschoben werden kann, bis der Anschlag erreicht ist. Das gibt der Handhabungsperson das taktile Zeichen, dass der Zusammenbau aus der jeweiligen Trokarhülse und dem entsprechenden Dorn mit der zutreffenden Relativlage dieser beiden Bauteile bewerkstelligt ist.

In einer weiteren Ausgestaltung der Erfindung ist ein Anschlag als vom Kopf vorspringender Stutzen ausgebildet, in den ein proximaler Bereich des Dornkörpers aufgenommen ist.

Diese Maßnahme hat bei relativ durchmesserstarken Trokardornen zum einen den Vorteil, dass ein solcher relativ bulkiger Trokardorn durch den Stutzen besonders fest und sicher gehalten wird. Da der Stutzen entsprechend durchmessergrößer ausgebildet sein muss, kann der Körper des Stutzens zugleich als ein solcher Anschlag ausgebildet sein, beispielsweise dessen distale Stirnkante.

In einer weiteren Ausgestaltung der Erfindung sind im Kopf Öffnungen ausgespart, in denen jeweils ein proximaler Endabschnitt eines Dornkörpers aufnehmbar ist.

Diese Maßnahme hat den erheblichen Vorteil, dass die Verbindung zwischen Kopf und Dornkörper besonders intensiv und somit stabil bewerkstelligt werden kann.

Bei Ausgestaltungen, bei denen die Dornkörper fest mit dem Kopf verbunden sind, können diese beispielsweise über Gewinde eingedreht, eingeklebt oder auch eingeschweißt werden. Bei den Ausgestaltungen mit auswechselbaren Dornkörpern stellen diese Öffnungen zugleich Führungen dar, um den auswechselbaren Schaft bzw. dessen distales Ende gezielt an beispielsweise eine Verrastung oder lösbare Kupplung heranzuführen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Ansicht eines ersten Ausführungsbeispiels eines erfindungsgemäßen Trokardornes mit zwei im Abstand zueinander angeordneten Dornkörpern, wobei diese unterschiedliche Durchmesser aufweisen,
- Fig. 2: einen abschnittsweisen Längsschnitt des Trokardornes von Fig. 1,
- Fig. 3: perspektivisch zwei Trokarhülsen, die auf die beiden Dornkörper, wie sie in Fig. 1 dargestellt sind, aufgeschoben werden können,
- Fig. 4: eine perspektivische Ansicht eines Zusammenbaus aus dem Trokardorn von Fig. 1 und den beiden Trokarhülsen von Fig. 3 zu dem resultierenden Trokar,
- Fig. 5: eine Draufsicht auf die proximale Stirnfläche des Trokardornes von Fig. 1,
- Fig. 6: eine entsprechende Draufsicht eines zweiten Ausführungsbeispiels eines Trokardornes mit drei Dornkörpern,
- Fig. 7: eine Seitenansicht eines dritten Ausführungsbeispiels eines Trokardornes in ähnlicher Ausgestaltung wie der Trokardorn von Fig. 1 mit der Ausnahme, dass einer der beiden Dornkörper lösbar am Kopf angebracht ist,
- Fig. 8: eine Darstellung des Trokarkörpers von Fig. 7 teilweise im Längsschnitt mit einem vom Kopf gelösten Dornkörper,
- Fig. 9: eine Draufsicht auf die proximalseitige Deckfläche des Kopfes eines alternativen, nicht erfindungsmäßen Trokardorns mit einem Verstellmechanismus zum Verändern des Abstandes von zwei an Dornkörpern untereinander in einem Zustand mit einem Minimalabstand A der beiden Dornkörper,
- Fig. 10: eine Seitenansicht des Trokardorns von Fig. 9,
- Fig. 11: eine der Darstellung von Fig. 9 vergleichbare Draufsicht, in dem der Abstand der beiden Dornkörper auf einen maximalen Abstand vergrößert worden ist, und
- Fig. 12: eine der Fig. 10 entsprechende Seitenansicht des Trokardornes in diesem maximal beabstandeten Zustand.

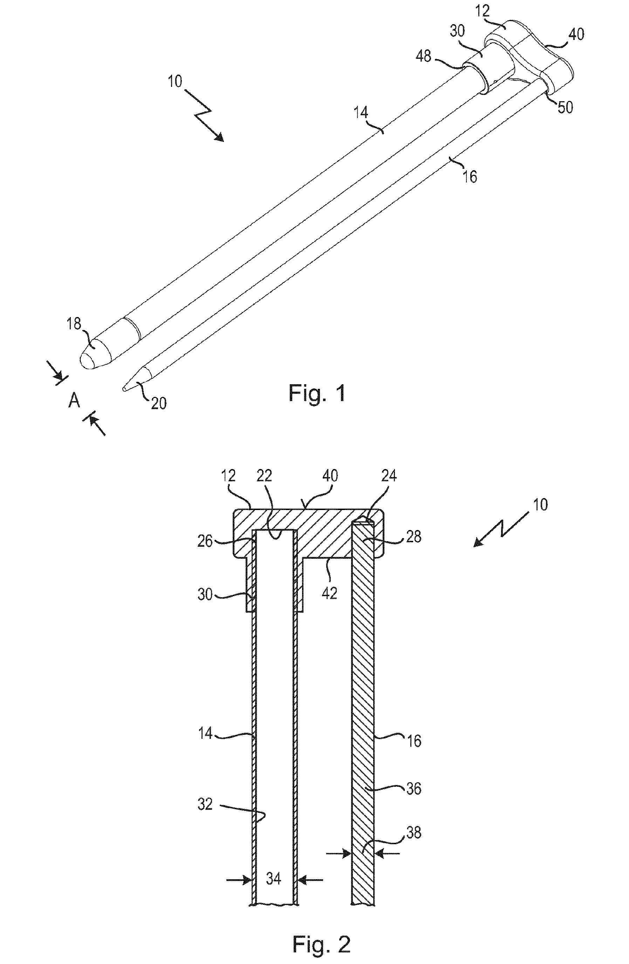

Ein in den Fig. 1 bis 5 dargestelltes erstes Ausführungsbeispiel zeigt einen Trokardorn, der in seiner Gesamtheit mit der Bezugsziffer 10 bezeichnet ist.

Der Trokardorn 10 weist einen Kopf 12 auf, von dem sich ein erster Dornkörper 14 und ein zweiter Dornkörper 16 gleichsinnig gerichtet und parallel zueinander wegerstrecken Jeder der Dornkörper 14 bzw. 16 weist distalseitig eine Spitze 18 bzw. 20 auf. Proximalseitig sind die Dornkörper 14 und 16, wie das insbesondere aus der Schnittdarstellung von Fig. 2 ersichtlich ist, in Öffnungen 22 bzw. 24 des Kopfes 12 aufgenommen. Dabei steckt ein proximaler Endabschnitt 26 des ersten Dornkörpers 14 in einer ersten Öffnung 22 im Kopf 12 und ist dort fest verankert. Der Außendurchmesser des ersten Dornkörpers 12 entspricht dabei dem lichten Innendurchmesser der Öffnung 22. Die Befestigung kann durch Verkleben, Verschweißen oder durch Fixierstifte erfolgen.

Vom Kopf 12 erstreckt sich nach distal ein Stutzen 30 fort, der die Öffnung 22 umrundet.

Dieser Stutzen 30 stellt eine zusätzliche Stütze und Führung für den relativ großen Dornkörper 14 auf, der einen Durchmesser 34 von 11 mm aufweist, der größer ist als der Durchmesser 38 des zweiten Dornkörpers 16, der 5,5 mm beträgt.

Wie aus der Schnittdarstellung von Fig. 2 zu erkennen, ist der erste Dornkörper 14 als ein Hohlkörper 32 ausgebildet.

Der zweite Dornkörper 16 mit dem geringeren Durchmesser 38 ist als massiver Stab 36 ausgebildet, der in die Öffnung 24 eingeschweißt ist.

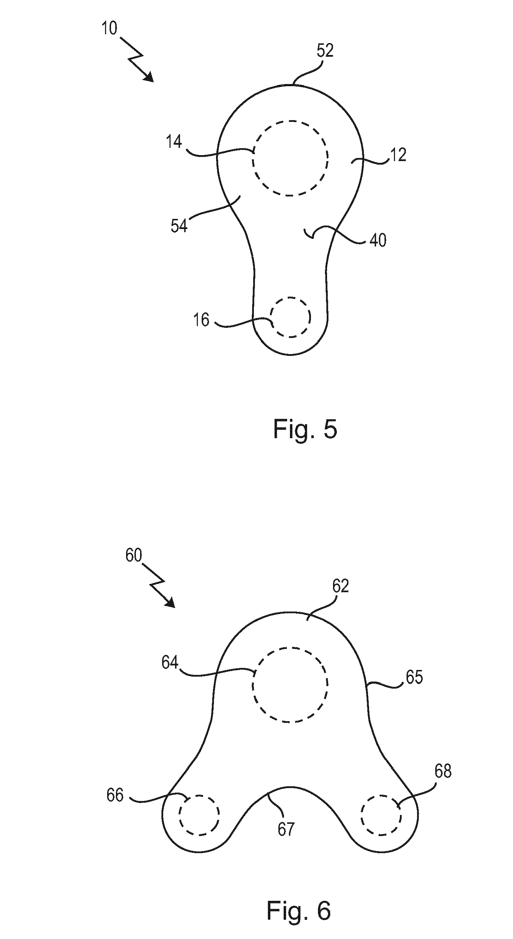

Der Kopf 12 weist eine ebene proximale Deckfläche 40 auf, wie das insbesondere aus der Darstellung von Fig. 2 und Fig. 5 ersichtlich ist. Die proximale Deckfläche 40 ist geschlossen und die beiden Dornkörper 14 und 16 erstrecken sich vom gegenüberliegenden distalen Ende 42 des Kopfes 12 weg. Die beiden Dornkörper 14 und 16 sind somit in einem Abstand A, wie das in Fig. 1 angedeutet ist zueinander angeordnet.

Aus Fig. 5 ist zu entnehmen, dass ein Umfangsprofil 52 der proximalen Deckfläche 40 einer Hüllkurve entspricht, die die beiden Dornkörper 14 und 16 mit unterschiedlichem Durchmesser umrundet.

Dadurch entsteht ein abgerundeter Körper 54, der besonders ergonomisch von Hand ergreifbar ist. Zusätzlich bildet die proximale Deckfläche 40 eine Angriffsfläche bei der Handhabung des Trokardornes 10, wie das nachfolgend noch beschrieben wird.

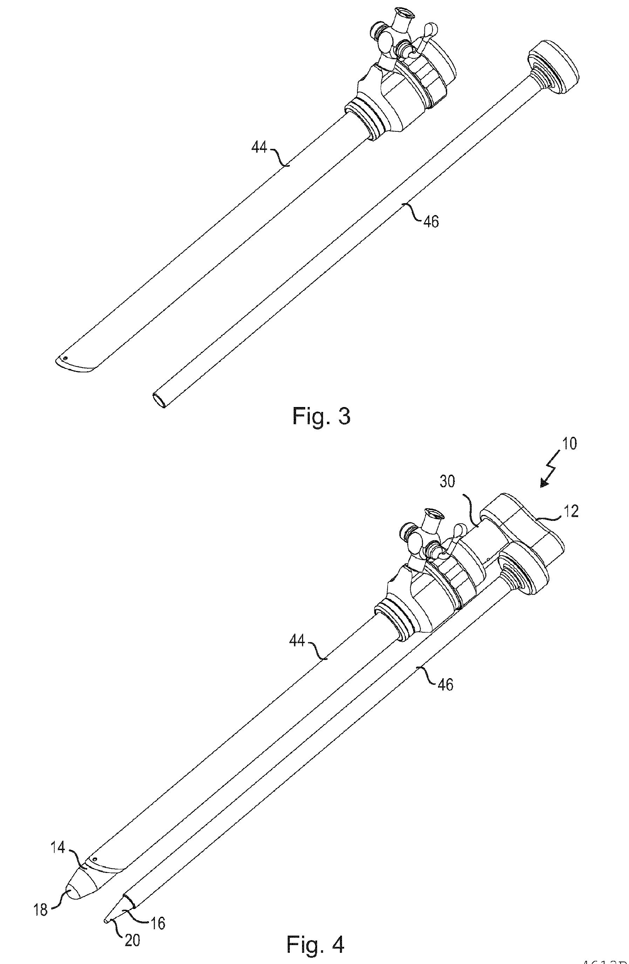

In Fig. 3 ist eine Trokarhülse 44 dargestellt, deren Hülsendurchmesser so ausgebildet ist, dass diese Trokarhülse auf den ersten Dornkörper 14 passend aufgeschoben werden kann.

Entsprechend ist die zweite Trokarhülse 46 so ausgebildet, dass sie passend auf den zweiten Dornkörper 16 aufgeschoben werden kann.

Dabei bildet die stirnseitige Ringfläche des Stutzens 30 ein Anschlag 48, bis zu dem die Trokarhülse 44 aufgeschoben werden kann. Der Bereich des distalen Endes 42 um den zweiten Dornkörper 16 herum bildet dann einen Anschlag 50 für die zweite Trokarhülse 46.

In Fig. 4 ist nun ein Zusammenbau dargestellt, bei dem die Trokarhülse 44 auf den ersten Dornkörper 14 und die Trokarhülse 46 auf den zweiten Dornkörper 16 aufgeschoben ist.

In dem in Fig. 4 dargestellten Bauzustand ist nunmehr ein Trokar realisiert, der über die beiden Dornkörper 14 und 16 zwei parallel und in einem bestimmten Abstand zueinander angeordnete Trokarhülsen 44 und 46 trägt.

Wie aus der Darstellung von Fig. 4 ersichtlich ist, ragen die Spitzen 18 und 20 der Dornkörper 14 und 16 distalseitig über die Trokarhülsen 44 und 46 hinaus.

Bei der Handhabung kann nun dieser Zusammenbau ergriffen werden und beispielsweise bei einem laparoskopischen Eingriff an einer oder zwei Inzisionen an der Bauchdecke angesetzt werden, wobei die beiden Spitzen 18 und 20 an diesen Inzisionen angesetzt werden.

Nunmehr kann durch Drücken auf die proximale Deckfläche 40 der Trokar durch die Bauchdecke hindurchgeschoben werden. Nach dem Setzen wird der Trokardorn 10 abgezogen und die beiden Trokarhülsen 44 und 46 verbleiben in der Bauchdecke und zwar in der gewünschten Ausrichtung und vor allem in dem gewünschten Abstand A zueinander. Nunmehr kann durch die Trokarhülse 44 und 46 der gewünschte minimal invasive Eingriff durchgeführt werden.

In Fig. 6 ist eine Draufsicht auf einen Kopf 62 eines zweiten Ausführungsbeispiels eines in seiner Gesamtheit mit der Bezugsziffer 60 bezeichneten Trokardorns dargestellt. Es ist ersichtlich, dass im Kopf 62 ein erster Dornkörper 64 aufgenommen ist, der in Größe und Durchmesser etwa dem ersten Dornkörper 14 des ersten Ausführungsbeispieles entspricht.

Abweichend zum zuvor beschriebenen ersten Ausführungsbeispiel sind jedoch hier ein zweiter Dornkörper 66 und ein dritter Dornkörper 68 vorhanden, wobei die beiden Dornkörper 66 und 68 einen geringeren Durchmesser als der Dornkörper 64 aufweisen.

Der Abstand des zweiten Dornkörpers 66 vom ersten Dornkörper 64 sowie der Abstand des dritten Dornkörpers 68 vom ersten Dornkörper 64 ist etwa gleich groß, dieser Abstand entspricht auch etwa dem Abstand vom zweiten und dritten Dornkörper 66 bzw. 68 untereinander. Das Umfangsprofil 65 des Kopfes 62 ist wieder als eine Art Umhüllungslinie der drei Dornkörper 64 bis 68 anzusehen und resultiert wiederum in einem ergonomisch abgerundeten Körper 67.

Ein solcher Körper ist sicher und ergonomisch von einer Hand ergreifbar, so dass ein solcher Trokardorn 60 mit auf den drei Dornkörpern 64 bis 68 aufgeschobenen Trokarhülsen sicher gehandhabt werden kann. Damit können dann drei Trokarhülsen in einem ganz bestimmten Abstand und in einer ganz bestimmten geometrischen Anordnung zueinander, hier auf den Ecken eines etwa gleichschenkligen Dreiecks liegend, im Körper gesetzt werden.

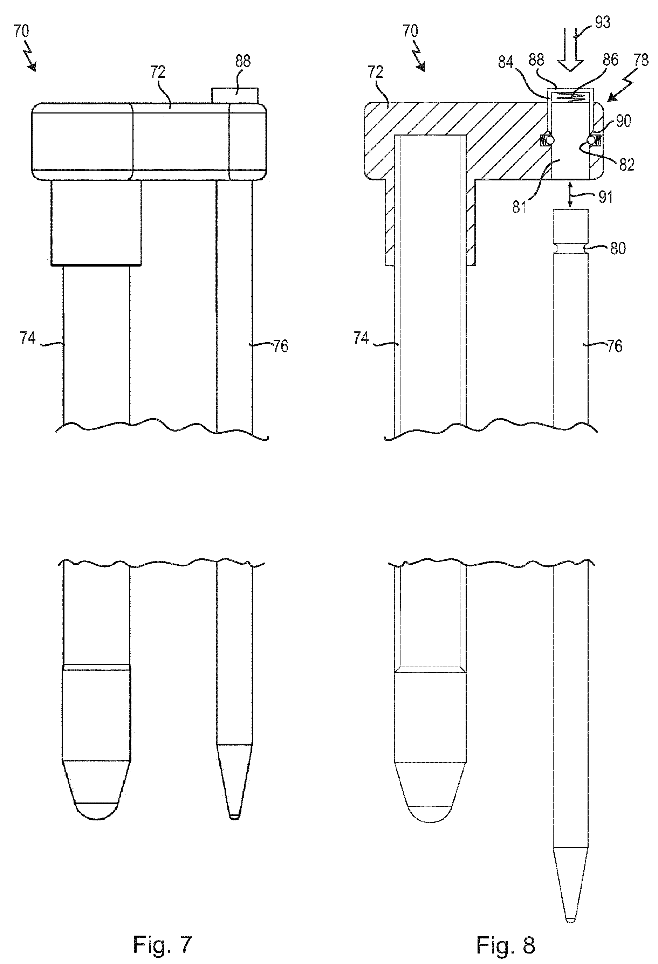

Ein in den Fig. 7 und 8 dargestelltes drittes Ausführungsbeispiel eines Trokardornes ist in seiner Gesamtheit mit der Bezugsziffer 70 bezeichnet.

Der Trokardorn 70 ist in seinen Grundelementen ähnlich aufgebaut wie der Trokardorn 10, d.h. er weist einen Kopf 72 auf, an dem ein erster Dornkörper 74 und ein zweiter durchmessergeringerer Dornkörper 76 angebracht ist.

Somit ist die grundsätzliche Konstruktion von Kopf und den beiden Dornkörpern 74 und 76 gleich wie beim Trokardorn 10.

Im Unterschied zum ersten Ausführungsbeispiel ist der zweite Dornkörper 76 lösbar am Kopf 72 angebracht. Dazu ist im Kopf 72 bzw. zweiten Dornkörper 76 eine Verrastung 78 vorgesehen.

Diese Verrastung 78 weist am zweiten Dornkörper 76 eine umfängliche Nut 80 auf.

In der Öffnung 81 im Kopf 72, in die der distale Endbereich des zweiten Dornkörpers 76 eingeschoben werden kann, wie das durch den Pfeil 91 angedeutet ist, sind mehrere federbelastete Kugeln 82 angeordnet.

Diese sitzen in seitlichen Ausnehmungen und werden in Richtung des Innenraumes der Öffnung 81 durch die entsprechenden Federn gedrückt. Die Lage ist dabei so, dass bei vollständig in die Öffnung 81 eingeschobenem zweitem Dornkörper 76 die Kugeln 82 in die Nut 80 eintreten können. Ein Betätigungselement 84 dient dazu, diese Kugelverrastung zu lösen.

Dazu kann das Betätigungselement 84 gegen die Kraft einer Feder 86, wie das durch den Pfeil 93 angedeutet ist, in Richtung auf die Kugeln 82 zu bewegt werden. Eine distale umlaufende Kante 90 des Betätigungselements 84 ist angeschrägt, so dass ein Bewegen des Betätigungselements 84 in Richtung des Pfeiles 93 entgegen der Kraft der Feder 86 die Kugel 82 radialseitig ausdrückt, so dass dann die Verrastung 78 gelöst ist.

Am oberen proximalen Ende ist das Betätigungselement als ein Knopf 88 ausgebildet, der über die distale Deckfläche des Kopfes 72 hinausragt, wie das insbesondere aus Fig. 7 ersichtlich ist.

Zum Befestigen des zweiten Dornkörpers 76 im Kopf 72 wird dieser, wie in Fig. 8 ersichtlich einfach in die Öffnung 81 hineingeschoben, bis die Kugeln 82 in die Nut 80 eintreten.

Zum Lösen wird der Knopf 88 eingedrückt, dadurch rasten die Kugeln aus und der zweite Dornkörper 76 kann abgenommen werden.

Dadurch ist die Möglichkeit eröffnet, beim Trokardorn 70 den zweiten Dornkörper 76 auszuwechseln, beispielsweise durch einen Trokardorn anderer Querschnittsgeometrie oder mit einer anderen Spitzencharakteristik oder dergleichen.

Im Prinzip ist es auch möglich, den ersten Dornkörper 74, falls das gewünscht ist, entsprechend lösbar auszugestalten.

Dadurch könnte dann auch der erste Dornkörper 74 ausgewechselt werden, so dass dann je nach Wahl nur einer der beiden oder auch alle beide Dornkörper 74 und 76 auswechselbar wären.

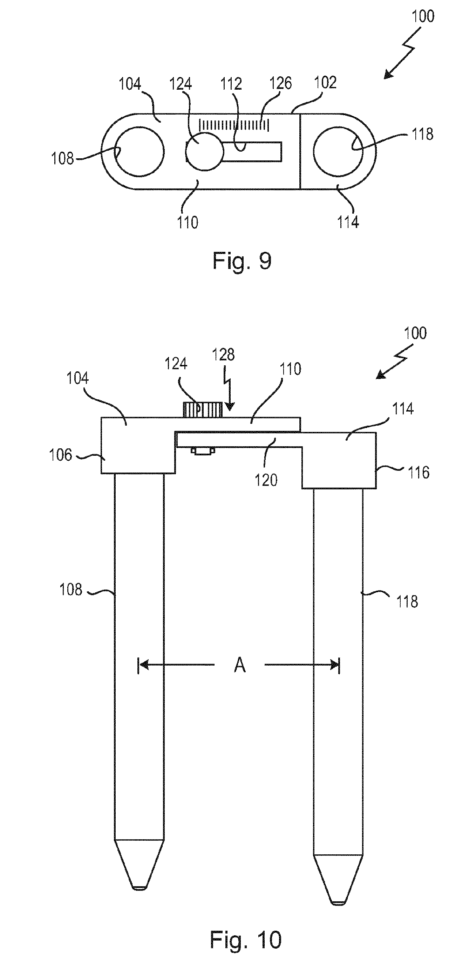

Bei dem in den Fig. 9 bis 12 dargestellten nicht erfindungsgemäßen Trokardorn ist dieser in seiner Gesamtheit mit der Bezugsziffer 100 bezeichnet. Diese alternative Variante zeigt keine Hüllkurve, die Dornkörper mit unterschiedlichem Durchmesser umrundet, dient jedoch zur Erläuterung des Verstellmechanismus, über den der Abstand zwischen benachbarten Dornkörpern (Anspruch 4) veränderbar ist.

Der Trokardorn 100 weist einen Kopf 102 auf, der aus einem ersten Teilstück 104 und einem zweiten Teilstück 114 zusammengesetzt ist.

Das erste Teilstück 104 weist einen nach distal vorspringenden Stutzen 106 auf, in dem ein erster Dornkörper 108 eingesetzt ist. Etwa im rechten Winkel dazu erstreckt sich eine ebene Lasche 110 vom Stutzen 106 weg, in der ein Schlitz 112 ausgespart ist.

Etwa spiegelbildlich erstreckt sich vom zweiten Teilstück 114 ebenfalls nach distal ein Stutzen 116 weg, in dem ein zweiter Dornkörper 118 aufgenommen ist.

In dieser Variante ist der Durchmesser von erstem Dornkörper 108 und zweitem Dornkörper 118 gleich groß. Auch vom zweiten Teilstück 114 erstreckt sich eine Lasche 120 weg, die einen Schlitz 122 aufweist.

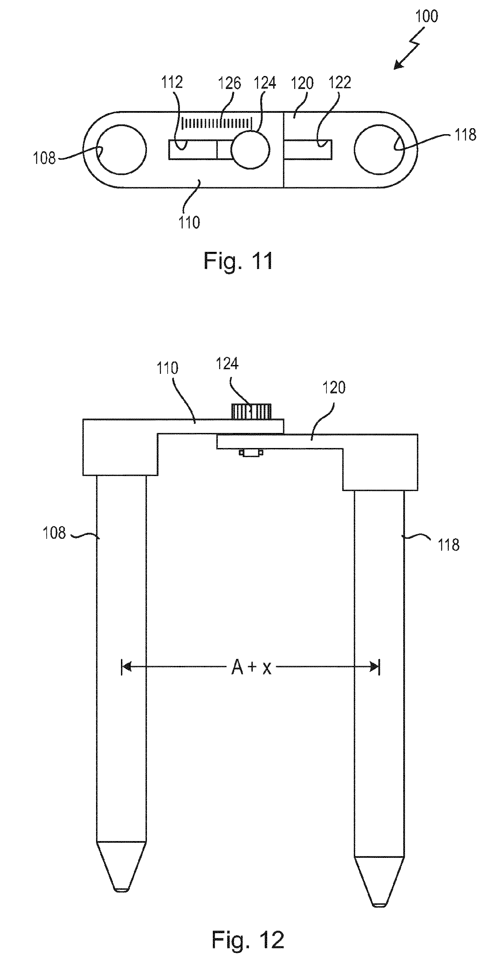

Im montierten Zustand, wie das insbesondere aus Fig. 9 und 11 hervorgeht, sind die beiden ebenen Laschen 110 und 120 so übereinandergelegt, dass deren Schlitze 112 und 122 übereinanderliegen.

Durch beide Schlitze 112, 122 hindurch reicht eine Knebelschraube 124, die dazu dient, diese beiden Teilstücke 104, 114 fest miteinander zu verbinden. Aus der Draufsicht von Fig. 9 ist ersichtlich, dass auf der Oberseite der Lasche 110 eine Skala 120 angeordnet ist.

Diese Bauelemente, also Laschen 110, 120, Schlitze 112, 122 und Knebelschraube 124 stellen insgesamt einen Verstellmechanismus 128 dar. Über diesen Verstellmechanismus 128 kann der Abstand A zwischen den beiden Dornkörpern 108 und 118 verändert werden.

In der Darstellung von Fig. 9 und 10 ist der Abstand A das Mindestmaß des Abstandes. D.h. die beiden Laschen 110 und 120 sind maximal aufeinander zu bewegt und über die Knebelschraube 124 aneinander fixiert.

Durch Lösen der Knebelschraube 124 können die beiden Teilstücke 104 und 114 somit die daran befestigten Dornkörper 108, 118 durch Auseinanderziehen im Abstand verändert werden, wobei in den Fig. 11 und 12 nunmehr der maximale Abstand A+x dargestellt ist. Insbesondere aus Fig. 11 ist zu erkennen, dass die Knebelschraube 124 in dieser Stellung am rechten Ende des Schlitzes 112 anstößt und zugleich am linken Ende des darunter angeordneten Schlitzes 122.

Insgesamt gesehen kann somit der Abstand zwischen den beiden Dornkörpern 108, 118 zwischen dem in Fig. 10 dargestellten Abstand A und dem in Fig. 12 dargestellten Abstand A+x variiert werden.

Die Skala 120 gibt der Handhabungsperson ein Maß für die Verschiebung x.

Es ist auch denkbar, dass bei gelöster Knebelschraube 124 nicht nur eine Bewegung der beiden Teilstücke 104, 114 längs der Längserstreckung der Schlitze 112, 122 möglich ist, sondern dass diese auch gegeneinander ab- oder angewinkelt werden können und in angewinkeltem Zustand fixiert werden.

Es kann auch vorgesehen sein, die in Fig. 7 und 8 dargestellte Variante der Lösbarkeit eines Dornkörpers auch bei dem vierten Ausführungsbeispiel vorzusehen, so dass dann eine besonders hohe Flexibilität, was die Anordnung des Abstandes und die Ausgestaltung der einzusetzenden Dornkörper entspricht.

## Patentansprüche

1. Trokardorn mit einem langerstreckten schaftartigen Dornkörper (14, 64, 74), dessen distales Ende mit einer Spitze (18, 20) versehen ist, und dessen proximales Ende einen Kopf (12, 62, 72) aufweist,
wobei am Kopf (12, 62, 72) zumindest ein zweiter langerstreckter schaftartiger Dornkörper (16; 66, 68, 76) derart angebracht ist, dass sich die Dornkörper in einem Abstand (A) zueinander vom Kopf wegerstrecken, wobei der Kopf (12) eine proximale Deckfläche (40) aufweist, und sich die schaftartigen Dornkörper vom distalen Ende (42) des Kopfes (12) wegerstrecken, wobei der Kopf (12, 62, 72), auf die proximale Deckfläche (40) gesehen, als abgerundeter Körper (54) ausgebildet ist, **dadurch gekennzeichnet dass** ein Umfangsprofil (52, 65) der Deckfläche (40) einer Hüllkurve entspricht, die die Dornkörper (14, 16; 64, 66, 68) mit unterschiedlichem Durchmesser umrundet.

2. Trokardorn nach Anspruch 1, **dadurch gekennzeichnet, dass** zumindest einer der schaftartigen Dornkörper (76) lösbar am Kopf (72) angebracht ist.

3. Trokardorn nach Anspruch 2, **dadurch gekennzeichnet, dass** der zumindest eine lösbare Dornkörper (76) mit dem Kopf (72) über eine Verrastung (78) lösbar verbindbar ist, wobei am Kopf (72) ein Betätigungselement (84) angeordnet ist, durch das die Verrastung (78) zwischen Kopf (72) und dem Dornkörper (76) lösbar ist.

4. Trokardorn nach einem der Ansprüche 1 bis 3, **dadurch gekennzeichnet, dass** an der Seite des Kopfes (12), von der die schaftartigen Dornkörper (14, 16) vorstehen, Anschläge (48, 50) angeordnet sind, die eine Einschubtiefe des jeweiligen Dornkörpers (14, 16) in eine Trokarhülse (44, 46) begrenzen.

5. Trokardorn nach Anspruch 4, **dadurch gekennzeichnet, dass** ein Anschlag (48) als vom Kopf (12) vorspringender Stutzen (30) ausgebildet ist, in den ein proximaler Bereich eines Dornkörpers (14) aufgenommen ist.

6. Trokardorn nach einem der Ansprüche 1 bis 5, **dadurch gekennzeichnet, dass** im Kopf (12, 72) Öffnungen (22, 24; 81) ausgespart sind, in die jeweils ein proximaler Endabschnitt (26, 28) eines Dornkörpers (14, 16) aufnehmbar ist.

## Claims

1. A trocar mandrel comprising an elongated shaft-like mandrel body (14, 64, 74), the distal end of which is provided with a tip (18, 20), and the proximal end of which comprises a head (12, 62, 72),
wherein at least one second elongated shaft-like mandrel body (16; 66, 68, 76) is attached to the head (12, 62, 72) in such a way that the mandrel bodies extend away from the head at a distance (A) with respect to one another, wherein the head (12) comprises a proximal cover surface (40), and that the shaft-like mandrel bodies extend away from the distal end (42) of the head (12), wherein the head (12, 62, 72), seen towards the proximal cover surface (40), is arranged as a rounded body (54), **characterized in that** a circumferential profile (52, 65) of the cover surface (40) corresponds to an envelope curve that encircles the mandrel bodies (14, 16; 64, 66, 68) at a different diameter.

2. The trocar mandrel according to claim 1, **characterized in that** at least one of the shaft-like mandrel bodies (76) is attached to the head (72) in a releasable fashion.

3. The trocar mandrel according to claim 2, **characterized in that** the at least one releasable mandrel body (76) is arranged to be connected to the head (72) in a releasable fashion via a locking feature (78), wherein an actuation element (84) is arranged at the head (72), wherein the locking feature (78) between the head (72) and the mandrel body (76) is releasable by the actuation element (84).

4. The trocar mandrel according to any of the claims 1 - 3, **characterized in that** limit stops (48, 50) are arranged at the side of the head (12) from which the shaft-like mandrel bodies (14, 16) protrude, wherein the limit stops (48, 50) delimit an insertion depth of the respective mandrel body (14, 16) in a trocar sleeve (44, 46).

5. The trocar mandrel according to claim 4, **characterized in that** a limit stop (48) is arranged as a stud (30) protruding from the head (12), wherein a proximal portion of a mandrel body (14) is received in the stud (30).

6. The trocar mandrel according to any of the claims 1 - 5, **characterized in that** openings (22, 24; 81) are recessed in the head (12, 72), wherein a proximal end portion (26, 28) of a mandrel body (14, 16) is arranged to be received in the respective openings (22, 24; 81).

## Revendications

1. Goujon de trocart comprenant un corps de goujon allongé de type tige (14, 64, 74), dont l'extrémité distale est pourvue d'une pointe (18, 20) et dont l'extrémité proximale présente une tête (12, 62, 72),
au moins un deuxième corps de goujon allongé de type tige (16 ; 66, 68, 76) étant monté sur la tête (12, 62, 72) de telle sorte que les corps de goujon s'étendent à une distance (A) l'un de l'autre à l'écart de la tête, la tête (12) présentant une surface de recouvrement proximale (40) et les corps de goujon de type tige s'étendant à l'écart de l'extrémité distale (42) de la tête (12), la tête (12, 62, 72), vu sur la surface de recouvrement proximale (40), étant réalisée sous forme de corps arrondi (54), **caractérisé en ce qu'**un profil périphérique (52, 65) de la surface de recouvrement (40) correspond à une courbe d'enveloppement qui entoure les corps de mandrin (14, 16 ; 64, 66, 68) avec un diamètre différent.

2. Goujon de trocart selon la revendication 1, **caractérisé en ce qu'**au moins l'un des corps de goujon de type tige (76) est monté de manière amovible sur la tête (72).

3. Goujon de trocart selon la revendication 2, **caractérisé en ce que** l'au moins un corps de goujon amovible (76) peut être connecté de manière amovible à la tête (72) par le biais d'un encliquetage (78), un élément d'actionnement (84) étant disposé au niveau de la tête (72), lequel permet de desserrer l'encliquetage (78) entre la tête (72) et le corps de goujon (76).

4. Goujon de trocart selon l'une quelconque des revendications 1 à 3, **caractérisé en ce que**, du côté de la tête (12) depuis lequel les corps de goujon de type tige (14, 16) font saillie sont disposées des butées (48, 50) qui limitent une profondeur d'enfoncement de chaque corps de goujon (14, 16) dans une douille de trocart (44, 46).

5. Goujon de trocart selon la revendication 4, **caractérisé en ce qu'**une butée (48) est réalisée sous forme de raccord (30) faisant saillie depuis la tête (12), dans lequel est reçue une région proximale d'un corps de goujon (14).

6. Goujon de trocart selon l'une quelconque des revendications 1 à 5, **caractérisé en ce que** des ouvertures (22, 24 ; 81) sont réalisées dans la tête (12, 72), dans lesquelles ouvertures peut à chaque fois être reçue une portion d'extrémité proximale (26, 28) d'un corps de goujon (14, 16).
